# EUROPEAN PATENT SPECIFICATION

(11) **EP 1 410 760 B1**
(45) Date of publication and mention of the grant of the patent: **04.03.2009**
(21) Application number: 01936464.5
(22) Date of filing: 30.05.2001
(51) Int. Cl.: A61B 5/117, G06K 9/00, G09F 3/00

(54) **SYSTEM AND ELECTRONIC DEVICE FOR IDENTIFYING NEONATES**
SYSTEM UND ELEKTRONISCHES GERÄT ZUR IDENTIFIKATION VON NEUGEBORENEN
SYSTEME ET DISPOSITIF ELECTRONIQUE D'IDENTIFICATION DE NOUVEAU-NES

(30) Priority: 30.05.2000 ES 200001375
(43) Date of publication of application: 21.04.2004
(73) Proprietor: Identificacion y Custodia Neonatal, S.A., 28707 S. Sebastian de los Reyes (ES)
(72) Inventor: HERREROS RODRIGUEZ, Carlos, Javier, E-28707 San Sebastian de los Reyes (ES)
(74) Representative: Manzano Cantos, Gregorio
(86) International application number: PCT/ES2001/000219
(87) International publication number: WO 2001/091641

(56) References cited:
- EP-A2- 0 460 533
- EP-A2- 0 750 880
- WO-A2-99/47036

## Description

According to the title, the object of the invention is to develop a system with its corresponding electronic equipment for the identification of neonates and whose final purpose is to develop a process permitting the management of neonate information and identify them to faithfully assure the one-to-one relation between mothers and their neonates.

Following the invention, the identification system as claimed in claim 1 is based on the capture of the mother and neonate's fingerprints plus the identification methods based on coders to assure the safe opening of the electronic file containing the fingerprints of both, without duplications, omissions or errors. Coders are worn by the mothers on their wrist and by neonate on the ankle during their stay in the hospital centre. But safe electronic files may also be opened from the biometric reading directly from the mother's fingerprints or from other known available biometric parameters such as iris reading, palm reading or similar.

The system in accordance with the invention must assure that a same register includes the mother's fingerprint (index finger of the right hand) and the neonate's fingerprints (right index and middle fingers) (if other fingers are used, it is indicated). This register is going to be identified by means of the code from a process called "Neonatal Custody Coded System", that will be represented as a bar code in the coders. The reading of this code will allow access to the register, if it already exists, or create it in this moment if it does not yet exist. Other factors, like the patient's name or DNI (National Identification Card) number have a secondary value in the process and will never be the base to relate the mother with her neonates.

To assure this one-to-one relation between the mother and neonate's fingerprints two methods may be used:
1.- If the mother's fingerprint has been previously captured (before entering the maternity ward/operating theatre), a verification should be made before taking the prints of the neonate.
2.- If the mother's fingerprint has not been previously captured, both fingerprint captures (mother and child) should be taken consecutively once birth has taken place.

Likewise, the system in accordance with the invention additionally foresees the possibility of printing the prints captured from the mother and her neonate at a high resolution in the required different medical and civil document forms.

### BACKGROUND OF THE INVENTION

As background of the invention, other processes from the same inventor exist in which on the one hand, and according to Patent No. ES-A-2 155 757 "AMBIVALENT ELECTRO-COMPUTERISED UNIT TO OBTAIN NEONATE FINGERPRINTS", a digital capture computer system of the neonate's fingerprint is disclosed, as well as EP-A-1 111 568 "CODED MEANS AND SYSTEM FOR NEONATAL CARE", a coder system permanently identifying both civil, hospital and professional documentation of the mother and neonate, whether the mother's identity is known or not or if a correct identification has been made.

The first electronic system, modified and improved in the system proposed by the invention and moreover related or shared by the patented method of neonate coders, constitutes an efficient, absolute and irrefutable system for control of the mother and her neonate.

### DESCRIPTION OF THE INVENTION.

The equipment according to a preferred embodiment of the invention will comprise a host computer which will permanently contain all the information generated and some 'portable equipment' responsible from capturing information in different places, wherever the mother and her neonate are to be found.

The basic equipment according to one embodiment of the invention will comprise a host computer unit and a 'portable unit'.

The 'host computer unit' according to the invention will basically comprise:
- A high performance computer and CD-ROM or equivalent equipment.
- A back-up system.
- A high-resolution printer.
- A device to capture the mother's fingerprint.
- A device to capture the baby's fingerprints.
- An optional ambivalent device to capture the prints of both (this will replace the aforementioned specific devices).
- A device to read the bar codes.
- An infrared device (IrDA), to communicate with the 'portable units'. (These devices may require in some cases the installation of PCI type cards or a determined connection method).

The portable 'equipment unit' according to one embodiment of the invention, will comprise the following components:
- A high performance portable terminal with touch screen or similar equipment.
- A device to capture the mother's fingerprint.
- A device to capture the baby's fingerprint.
- An optional ambivalent device to capture prints from both of them (It will replace the aforementioned specific devices).
- A device for reading the bar codes.
- An infrared device (IrDA) to communicate with the central 'host computer'. (These devices may require in some cases the installation of PCMCIA type cards or a determined connection mode.

A better idea of the features of the invention will be obtained below, referring to the drawing attached to this description, schematically and only as an example, representing the preferred and essential details of the invention.

IN THE DRAWINGS:
Figure 1 is a schematic view displaying the central 'host computer' unit of the equipment.
Figure 2 is a schematic view displaying the 'portable unit' of the equipment.

### PREFERRED EMBODIMENT OF THE INVENTION

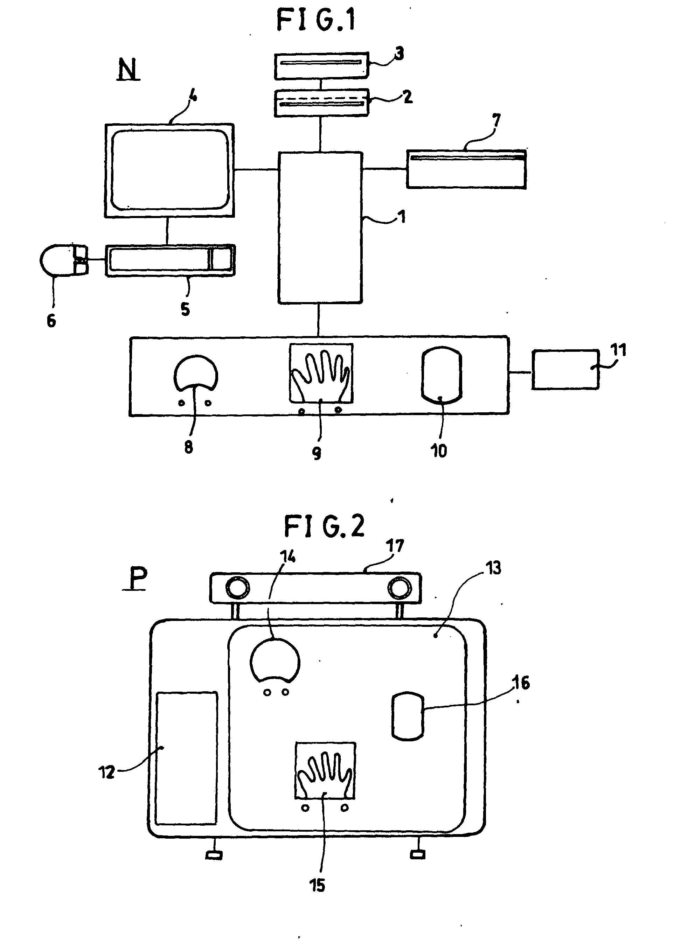

According to an interpretation of the preferred embodiment of the invention, the work bench proposed for the central unit or 'host computer' (N) shown by figure 1, will comprise the corresponding PC (1) with the aforementioned performances; CD-ROM (2) and corresponding back-up (3), also including a monitor (4), keyboard (5) and mouse (6); a device to capture the mother's fingerprint (8), a device to capture the baby's prints (9); an ambivalent device to capture the prints of both (8-9); an infrared device (11) to communicate with the 'portable unit' (P); the 'portable unit' (P) itself with its external supply and corresponding output for the high resolution printer (7).

Following the same criterion, the 'portable' unit (P) will comprise its corresponding 'portable PC' (12) with the same aforementioned characteristics and touch screen (13) or similar equipment, where the device to capture the mother's fingerprint (14) is incorporated; a device to capture the baby's print (15); an ambivalent device to capture both prints (14-15); a device to read the bar code (16) and the infrared device (IrDA) to communicate with the central 'host computer' unit (N).

The shell or surface containing the devices to capture the baby's prints (9); (15) will have sufficient width and flat undercutting without steps, so that the complete hand palm of the neonate may be placed to facilitate the print capture process. Likewise, to this neonate print capture device, specific silicon will be added, since it has been proved that it provides a better print quality.

Both for the 'host computer' (N) support design and for the camera housings of the 'portable' unit (P), the most ergonomically suitable and relevant for the application requirements will be developed

### PROCESS DEFINITION

The basic processes to be carried out with the neonate identification equipment object of the invention, are as follows:
**Process 1: Mother's Card Creation.**
   Equipment: Portable unit (P)
   Place: Before entering the maternity ward/operating theatre.
   - The bar code of the mother's wrist-strap coder is read.
   - The mother's fingerprint is taken (index finger of the right hand).
   - The matron is identified.
   - The portable equipment (P) may now be used by another matron in another delivery.
   NOTE: This process may be omitted or it is possible to be started in process 2.
**Process 2: Taking neonate's prints**
   Equipment: Portable unit (P)
   Place: operating theatre or incubator.
   - The bar code of the mother's wrist-strap coder is read.
   - If a register exists with this identification, the mother's fingerprint is taken (right index finger) to check that it corresponds with the print taken previously.
   - If there is not a register with this identification, it is created and the mother's print taken (right index finger). The matron is identified (Process 1).
   - After either of the two previous steps, the neonate's prints are taken (right index and middle fingers).
   - The register relating the mother's print with those of the neonate is closed.
**Process 3: Information Transfer between the 'portable' unit and the 'host computer'.**
   Equipment: Portable unit and 'host computer'.
   Place: Main station.
   - The bar code of the Manager-Matron is read.
   - The corresponding option is selected.
   - The data from the 'portable' (P) are transferred to the 'host computer' (N).
   - The 'portable' unit (P) data are deleted.
   - From the 'host computer' (N) it is verified that all the patient's data are available.
   - Otherwise, they are requested and may be introduced in this moment.
   - Printing options from the 'host computer' are offered.
**Process 4: Centre Release Report**
   Equipment: 'host computer' and portable unit.
   Place: Main station.
   - The bar code of the Manager-Matron is read.
   - The option 'release verification' is selected.
   - The existence of all the patient's data is checked. Otherwise, it is requested and introduced in this moment.
   - The option of data backup is again given to a 'portable' unit (N) so this operation is made from this equipment, to be transferred to the place where the patient is to be found.
   - The wrist coder of the patient is read from the 'host computer' (N) or 'portable' unit (P).
   - The two neonate prints are taken again (right index and middle fingers).
   - If a 'portable' unit (P) has been used, data backup will be to the 'host computer' (N).
   - From the 'host computer' (N), the report is printed with the mother's print and the four prints of her daughter or sun.
   NOTE: The latter two operations may be carried out directly from the portable unit.

According to the invention, in the current definition, the input point to the process is by means of the coder reading containing a bar code. Each one of these coders will be related to a patient during her stay in the Hospital or Maternity Clinic. Under normal conditions, the process will be in stand-by for the reading of this code to perform a specific operation.

According to the process of the invention, it should assure that these coders are maintained in an acceptable condition for their reading during the time a patient stays in hospital.

Likewise, according to the mentioned process, there is also a card containing a code identifying the Manager-Matron. Reading this code will allow access to the specific functions for this user (e.g.: information request; report preparation, etc.). This input mode also serves to cover exceptional situations, such as:
- The bar code of the mother's wrist strap cannot be read. In this case, the matron would enter in the system with her card and would locate the patient's record or create a new one if necessary.
- Verification of a previously taken mother's print cannot be made since she is not at this time in the same physical place as the neonate.

Once conveniently described the nature of the invention, it is recorded for pertinent purposes that it is not limited to the exact details of this explanation, but on the contrary, modifications will be introduced if considered relevant, provided they do not change the basic features thereof, which are claimed below:

## Claims

1. AN ELECTRONIC SYSTEM AND EQUIPMENT FOR NEONATE IDENTIFICATION, **CHARACTERISED by** comprising means for capturing in a same record both the mother's fingerprint and the neonate's fingerprint, means for electronically relating said fingerprints to a code, said code being represented as a bar code on a strap adapted to worn by the mother on her wrist and the neonate on the their ankle, and said fingerprints possibly also being electronically related to further biometric data , wherein reading of the code or of the biometric data permits access to a register.

2. AN ELECTRONIC SYSTEM AND EQUIPMENT FOR NEONATE IDENTIFICATION that permits access to a register according to claim 1, **CHARACTERISED by** comprising a central computer or 'host computer' (N) permanently containing all the information generated and portable equipment (P) responsible for capturing information in different places.

3. AN ELECTRONIC SYSTEM AND EQUIPMENT FOR NEONATE IDENTIFICATION according to claim 2, the 'host computer' equipment (N) being **CHARACTERISED by** comprising a PC (1) and CD-ROM (2), back-up (3); high resolution printer (7); a device to capture the mother's fingerprint (8); a device to capture the neonate fingerprint (9); an optional ambivalent device to capture both fingerprints (8-9); a device to read the bar codes (10) and an infrared device-IrDA- (11) to communicate with the portable unit (P).

4. AN ELECTRONIC SYSTEM AND EQUIPMENT FOR NEONATE IDENTIFICATION according to claims 2 and 3, the 'portable' units (P) being **CHARACTERISED by** comprising a high performance terminal (12) and touch screen (13); a device to capture the mother's fingerprint (14); a device to capture the neonate fingerprint (15), an optional ambivalent device to capture fingerprints from both (14-15); a device to read the bar codes (16) and an infrared device - IrDA- (17) to communicate with the 'host computer' equipment (N).

5. AN ELECTRONIC SYSTEM AND EQUIPMENT FOR NEONATE IDENTIFICATION according to claims 3 and 4, the devices to capture the neonate fingerprint (9), (15) or the optional ambivalent equipment, are **CHARACTERISED in that** the shell or surface containing said devices has sufficient width and a flat undercutting without steps to permit the complete palm of the neonate hand to be introduced.

6. AN ELECTRONIC SYSTEM AND EQUIPMENT FOR NEONATE IDENTIFICATION according to claims 1 and 5, in the devices (9) and (15) or the optional ambivalent equipment to capture the neonate's fingerprint, is **CHARACTERISED in that** specific silicon will be added to provide a better fingerprint quality.

## Patentansprüche

1. Elektronisches System und Gerät zur Identifikation von Neugeborenen, **dadurch gekennzeichnet, dass** es Mittel umfasst, um in derselben Aufzeichnung sowohl den Fingerabdruck der Mutter als auch den Fingerabdruck des Neugeborenen zu erfassen, Mittel zur elektronischen Zuordnung der Fingerabdrücke zu einem Code, wobei der Code als ein Strichcode auf einem Band dargestellt ist, angepasst, um von der Mutter an ihrem Handgelenk und von dem Neugeborenen an seinem Fußgelenk getragen zu werden, und wobei die Fingerabdrücke möglicherweise auch zu weiteren biometrischen Daten elektronisch zugeordnet sind, wobei das Lesen des Codes oder der biometrischen Daten Zugang zu einem Register gewährt.

2. Elektronisches System und Gerät zur Identifikation von Neugeborenen, dass nach Anspruch 1 Zugang zu einem Register gewährt, **dadurch gekennzeichnet, dass** es einen zentralen Computer oder "Host Computer" (N) umfasst, der dauerhaft alle gelieferten Informationen enthält und ein tragbares Gerät (P), das für die Erfassung von Information in verschiedenen Orten verantwortlicht ist.

3. Elektronisches System und Gerät zur Identifikation von Neugeborenen nach Anspruch 2, indem das "Host Computer"-Gerät **dadurch gekennzeichnet ist, dass** es einen PC (1) und CD-ROM (2), Back-up (3); einen hochauflösenden Drucker (7); eine Vorrichtung zur Erfassung des Fingerabdrucks (8) der Mutter; eine Vorrichtung zur Erfassung des Fingerabdrucks (9) des Neugeborenen; eine optionale, ambivalente Vorrichtung zur Erfassung beider Fingerabdrücke (8-9); eine Vorrichtung zum Lesen der Strichcodes (10) und eine Infrarotvorrichtung (11) IrDA zur Kommunikation mit der tragbaren Einheit (P)umfasst.

4. Elektronisches System und Gerät zur Identifikation von Neugeborenen nach den Ansprüchen 2 und 3, indem die "tragbaren" Einheiten (P) **dadurch gekennzeichnet sind, dass** sie ein Hochleistungsterminal (12) und einen Berührungsbildschirm (13); eine Vorrichtung zur Erfassung des Fingerabdrucks (14) der Mutter; eine Vorrichtung zur Erfassung des Fingerabdrucks (15) des Neugeborenen, eine optionale, ambivalente Vorrichtung zur Erfassung beider Fingerabdrücke (14-15); eine Vorrichtung zum Lesen der Strichcodes (16)und eine Infrarotvorrichtung (17) IrDA zur Kommunikation mit dem "Host Computer"-Gerät (N) umfassen.

5. Elektronisches System und Gerät zur Identifikation von Neugeborenen nach den Ansprüchen 3 und 4, indem die Vorrichtungen zur Erfassung des Fingerabdrucks (9), (15) des Neugeborenen oder das optionale, ambivalente Gerät **dadurch gekennzeichnet sind, dass** das Gehäuse oder die Oberfläche, welche die Vorrichtungen beinhaltet über eine ausreichende Breite und eine flache Hinterschneidung ohne Stufen hat, um die Einführung der gesamten Handfläche der Hand des Neugeborenen zu ermöglichen.

6. Elektronisches System und Gerät zur Identifikation von Neugeborenen nach den Ansprüchen 1 und 5, indem die Vorrichtungen (9) und (15) oder das optionale, ambivalente Gerät zur Erfassung des Fingerabdrucks des Neugeborenen **dadurch gekennzeichnet ist, dass** ein bestimmtes Silikon hinzugefügt wird, um eine bessere Qualität der Fingerabdrücke zu ermöglichen.

## Revendications

1. SYSTÈME ET EQUIPEMENT ELECTRONIQUE D'IDENTIFICATION DE NOUVEAU-NÉS, **CARACTÉRISÉ, en ce qu'**il comprend des moyens pour capturer dans un même enregistrement aussi bien l'empreinte digitale de la mère que l'empreinte digitale du nouveau-né, des moyens pour mettre en rapport électroniquement lesdites empreintes digitales avec un code, ledit code étant représenté comme un code à barres sur un bracelet destiné à être porté par la mère au poignet et par le nouveau-né à la cheville, et lesdites empreintes digitales étant également possiblement mis en rapport électroniquement avec d'autres données biométriques, la lecture du code ou des données biométriques permettant l'accès à un enregistrement.

2. SYSTÈME ET EQUIPEMENT ELECTRONIQUE D'IDENTIFICATION DE NOUVEAU-NÉS, qui permet l'accès à un enregistrement selon la revendication 1, **CARACTÉRISÉ en ce qu'**il comprend un ordinateur central ou « host computer » (N) contenant en permanence toute l'information générée et un équipement portable (P) chargée de capturer l'information à différents endroits.

3. SYSTÈME ET EQUIPEMENT ELECTRONIQUE D'IDENTIFICATION DE NOUVEAU-NÉS selon la revendication 2, l'équipement d'ordinateur central (N) étant **CARACTERISÉ en ce qu'**il comprend un PC (1) et un CD-ROM (2), secours informatique « back-up » (3); une imprimante haute résolution (7); un dispositif pour capturer l'empreinte digitale de la mère (8); un dispositif pour capturer l'empreinte digitale du nouveau-né (9); un dispositif facultatif ambivalent pour capturer les deux empreintes digitales (8-9); un dispositif pour lire les codes à barres (10) et un dispositif à infrarouge - IrDA- (11) pour communiquer avec l'unité portable (P).

4. SYSTÈME ET EQUIPEMENT ELECTRONIQUE D'IDENTIFICATION DE NOUVEAU-NÉS selon les revendications 2 et 3, les unités « portables » (P) étant **CARACTÉRISÉES en ce qu'**elles comprennent un terminal à haute performance (12) et un écran tactile (13); un dispositif pour capturer l'empreinte digitale de la mère (14); un dispositif pour capturer l'empreinte digitale du nouveau-né (15), un dispositif facultatif ambivalent pour capturer les empreintes des deux (14-15); un dispositif pour lire les codes à barres (16) et un dispositif à infrarouge - IrDA- (17) pour communiquer avec l'équipement d'ordinateur central (N).

5. SYSTÈME ET EQUIPEMENT ELECTRONIQUE D'IDENTIFICATION DE NOUVEAU-NÉS selon les revendications 3 et 4, les dispositifs pour capturer l'empreinte digitale du nouveau-né (9), (15) ou l'équipement facultatif ambivalent sont **CARACTERISÉS en ce que** la carcasse ou surface contenant lesdits dispositifs est suffisamment large et a une creusement sans échelons permettant l'introduction de la paume entière de la main du nouveau-né.

6. SYSTÈME ET EQUIPEMENT ELECTRONIQUE D'IDENTIFICATION DE NOUVEAU-NÉS selon les revendications 1 et 5, dans les dispositifs (9) et (15) ou l'équipement facultatif ambivalent pour capturer l'empreinte digitale du nouveau-né, est **CARACTERISÉ en ce que** l'on ajoutera de la silicone pour fournir une meilleure qualité de l'empreinte digitale.
